(19) Europäisches Patentamt / European Patent Office / Office européen des brevets

(11) **EP 3 263 062 A1**

(12) **EUROPEAN PATENT APPLICATION**
published in accordance with Art. 153(4) EPC

(43) Date of publication:
**03.01.2018 Bulletin 2018/01**

(51) Int Cl.:
**A61B 90/00** (2016.01)

(21) Application number: **16755220.7**

(22) Date of filing: **10.02.2016**

(86) International application number:
**PCT/JP2016/053971**

(87) International publication number:
**WO 2016/136473 (01.09.2016 Gazette 2016/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **26.02.2015 JP 2015036055**

(71) Applicant: **Olympus Corporation**
**Hachioji-shi, Tokyo 192-8507 (JP)**

(72) Inventors:
• **TAKAHASHI, Keigo**
  **Tokyo 192-8507 (JP)**
• **KISHI, Kosuke**
  **Tokyo 192-8507 (JP)**
• **YANAGIHARA, Masaru**
  **Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **MEDICAL MANIPULATOR SYSTEM**

(57) A manipulator system comprises a manipulator that includes a flexible manipulator 3 provided with an elongated flexible insertion portion 6, a movable portion 7 that is provided at a distal end of the insertion portion 6, and a driving portion 8 that is provided at a proximal end of the insertion portion 6 and that drives the movable portion 7; an operation input portion 2 that is operated by an operator and with which an operation instruction for the flexible manipulator 3 is input; a control unit 14 that controls the driving portion 8 on the basis of the operation instruction input via the operation input portion 2; an information input portion 12 with which treatment-site specifying information for specifying a treatment target by using the flexible manipulator 3 is input; and an information storage portion 13 that sets a control parameter for controlling the driving portion 8 on the basis of the treatment-site specifying information, wherein the control unit 14 controls the driving portion 8 on the basis of the treatment-site specifying information input via the information input portion 12 by using the control parameter set by the information storage portion 13.

FIG. 2

## Description

{Technical Field}

The present invention relates to a medical manipulator system.

{Background Art}

In the related art, there is a known technology whereby a control parameter of an electrical treatment tool, which is introduced into the body interior via a channel of a flexible endoscope, is changed in accordance with a bent state of the flexible endoscope (for example, see Patent Literature 1). In Patent Literature 1, the bent state of an insertion portion is detected on the basis of information detected by a sensor having a strain gauge that detects the amount of strain in the insertion portion and the tensile force in a wire that drives and bends a bending portion at the distal end of the insertion portion of the flexible endoscope.

{Citation List}

{Patent Literature}

{PTL 1} Publication of Japanese Patent No. 4580973

{Summary of Invention}

{Technical Problem}

However, with the method as in Patent Literature 1, in which the bent state of the insertion portion is detected on the basis of the tensile force in the wire, it is difficult to precisely detect the bent state when the wire is stretched and relaxed in association with the use thereof. In addition, with the method of Patent Literature 1, because disposing strain gauges in the individual portions of the insertion portion of the endoscope increases the diameter of the insertion portion and involves complicated signal processing, this is not desirable since the size and cost of the device are consequently increased.

The present invention has been conceived in light of the above-described circumstances, and an object thereof is to provide a medical manipulator system with which it is possible to precisely control a flexible manipulator by setting an appropriate control parameter without using a sensor.

{Solution to Problem}

In order to achieve the above-described object, the present invention provides the following solutions.

An aspect of the present invention is a medical manipulator system including: a flexible manipulator provided with an elongated flexible insertion portion, a movable portion that is provided at a distal end of the insertion portion, and a driving portion that is provided at a proximal end of the insertion portion and that drives the movable portion; an operation input portion that is operated by an operator and with which an operation instruction for the flexible manipulator is input; a drive control unit that controls the driving portion on the basis of the operation instruction input via the operation input portion; an information input portion with which treatment-site specifying information for specifying a treatment target by using the flexible manipulator is input; and an information control unit that sets a control parameter for controlling the driving portion on the basis of the treatment-site specifying information, wherein the drive control unit controls the driving portion on the basis of the treatment-site specifying information input via the information input portion by using the control parameter set by the information control unit.

With this aspect, when the treatment-site specifying information is input by the information input portion, the control parameter is set by the information control unit by using the input treatment-site specifying information. When the insertion portion of the flexible manipulator is inserted into the body to bring the movable portion at the distal end into a state in which the movable portion is in close contact with an affected portion and the operation instruction is input via the operation input portion, the drive control unit actuates, by using the set control parameter, the driving portion in accordance with the operation instruction input via the operation input portion. By doing so, the movable portion at the distal end of the flexible manipulator is driven, and thus, it is possible to treat the affected portion.

Because the insertion path of the flexible manipulator is substantially determined once the treatment site is specified, the bent shape of the flexible manipulator when inserted along this insertion path is also substantially determined. Thus, after the movable portion of the flexible manipulator has reached the treatment site, the movable portion can be operated without greatly changing the bent shape of the flexible manipulator.

Therefore, it is possible to unmistakably assume the bent shape of the flexible manipulator by using the information that specifies the treatment site, and it is possible to set a control parameter appropriate for the bent shape on the basis of the treatment-site specifying information. In other words, by using the appropriate control parameter, it is possible to accurately control the movable potion arranged at the distal end of the flexible manipulator by using the driving portion arranged at the proximal end. Thus, with such a medical manipulator system, it is possible to accurately control the movable portion by using an appropriate control parameter without using a sensor, avoid complicated signal processing, and reduce the size and the cost of the device.

In the above-described aspect, the information control unit may store the treatment-site specifying information and the control parameter in association with each other, and the drive control unit may control the driving portion on the basis of the treatment-site specifying information input via the information input portion by using the control parameter read out from the information control unit.

The above-described aspect may be provided with a guide member that has a greater rigidity than that of the insertion portion, and that guides the insertion portion.

By doing so, by inserting the guide member having a greater rigidity into the body of the patient and guiding the insertion of the flexible manipulator by the guide member, compared to the case where the flexible manipulator is inserted into the body alone, it is possible to enhance the control precision based on the selected control parameter by reducing a deviation of the insertion path.

In other words, when a soft flexible manipulator is inserted into the body cavity alone, it is inserted to the treatment site by twisting the insertion portion so as to follow the detailed shape of the inserted body cavity, but with the guide member having a greater rigidity than that of the insertion portion, it is inserted so as to reach the treatment site at a curvature according to its own rigidity so as to follow the rough shape of the body cavity or, in some cases, while changing the shape of the body cavity. Thus, by guiding the flexible manipulator so as to be inserted along the guide member that has been inserted in such a manner, because the flexible manipulator is bent in accordance with the shape of the guide member without being affected by the detailed shape of the body cavity, it is possible to suppress the changes in the insertion path and accurately control the driving portion by the drive control unit. By doing so, it is possible to form a flexible-manipulator shape in accordance with the control parameter that is appropriate for the bent shape that has been assumed and prepared in this system, and thus, it is possible to more accurately control the movable portion.

In the above-described aspect, the treatment-site specifying information may include a treatment-site name.

By doing so, it is possible for the operator to specify the treatment site only by inputting the treatment-site name, and thus, it is possible to read out the appropriate control parameter in a simple manner and control the flexible manipulator with high accuracy. In addition to the organ name, the treatment-site name may be symbols set to individual portions of an organ, diagrams indicating sites, or the like.

In the above-described aspect, the treatment-site name may be an organ name.

By doing so, in the case of a small organ, it is possible to specify the treatment site by using an easily recognized organ name, and it is possible to accurately control the driving portion by reading out the appropriate control parameter in a simple manner.

In the above-described aspect, the treatment-site name may be the organ name and a name of a section, the section that is divided in accordance with an insertion depth.

By doing so, in the case of a large organ, it is possible to more precisely specify the treatment site in the organ, and it is possible to more precisely set the control parameter in accordance thereto.

In the above-described aspect, the treatment-site specifying information may be a insertion length of the insertion portion into a body of a patient.

By doing so, it is possible to specify a position of the distal end of the flexible manipulator when inserted along an assumed path as the treatment site by using the insertion length of the insertion portion that is input to the information input portion.

In the above-described aspect, the treatment-site specifying information may be a treatment-site name and an insertion length of the insertion portion into a body of a patient.

By doing so, it is possible to specify the treatment site according to the treatment-site name, and further specify a precise area in the specified treatment site by the insertion length. For example, if the treatment site specified by using the treatment-site name is in a bent portion, when the insertion length changes, the bending angle thereof changes. Therefore, it is possible to correct the control parameter by using the insertion length, and thus, it is possible to more closely control the driving portion.

{Advantageous Effects of Invention}

The present invention affords an advantage in that it is possible to accurately control a manipulator by setting an appropriate control parameter without using a sensor.

{Brief Description of Drawings}

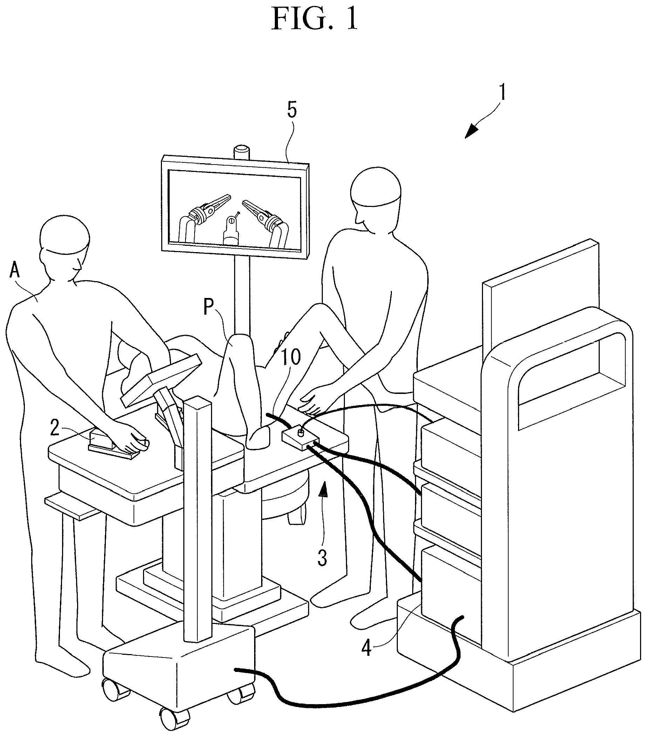

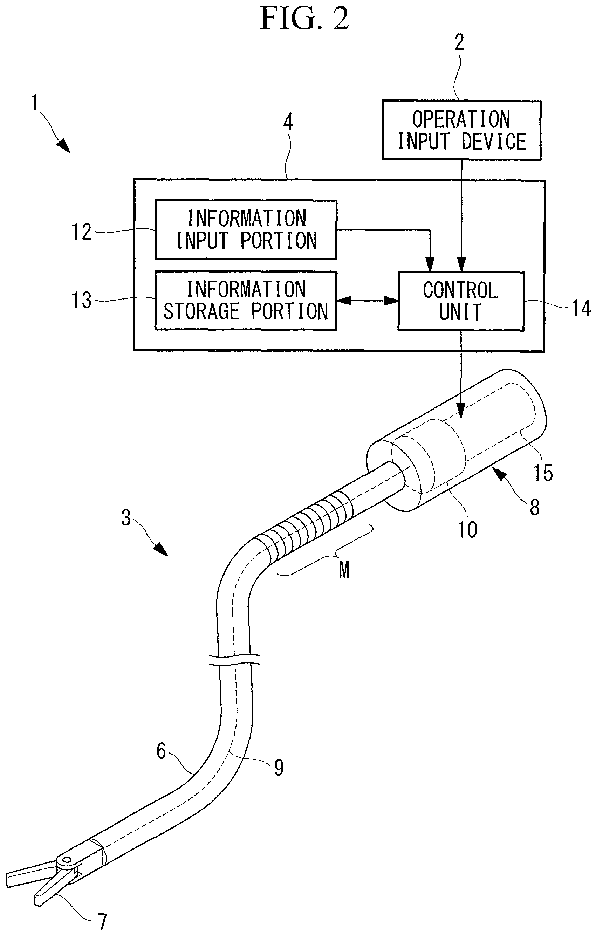

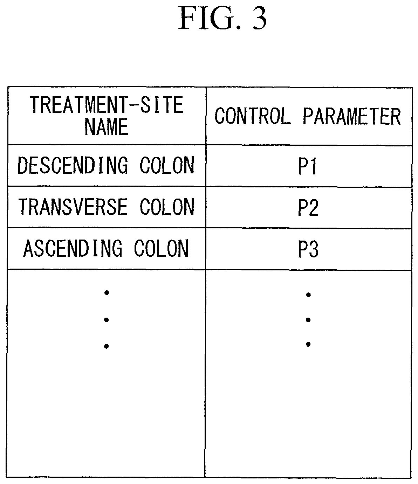

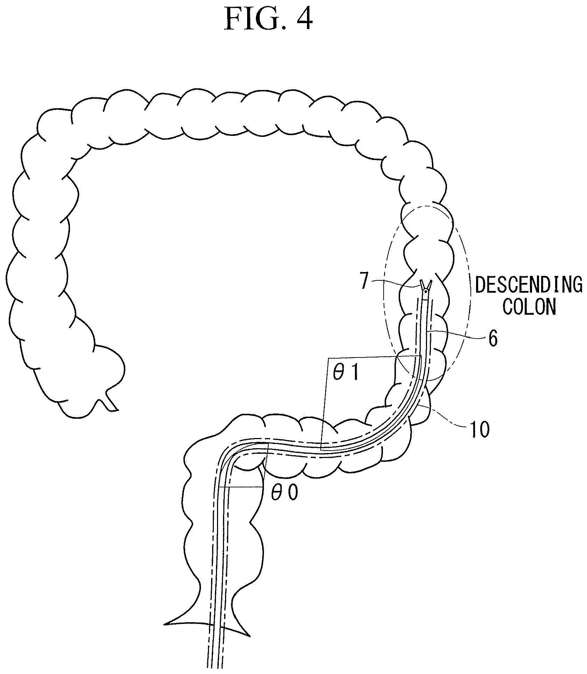

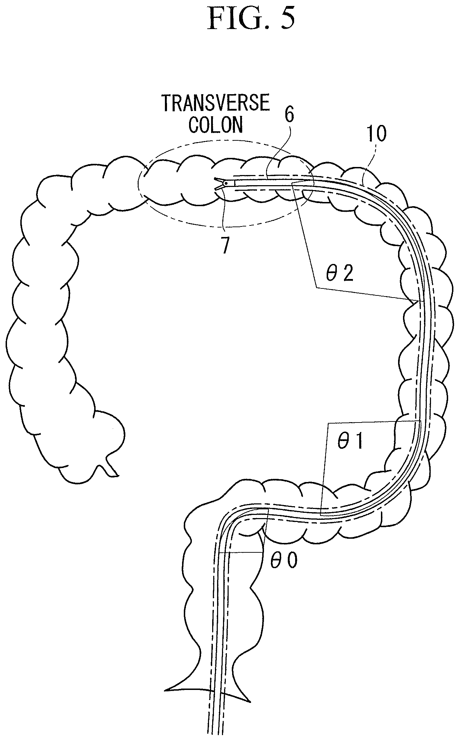

{Fig. 1} Fig. 1 is an overall configuration diagram showing a medical manipulator system according to an embodiment of the present invention.
{Fig. 2} Fig. 2 is a perspective view showing a flexible manipulator provided in the medical manipulator system in Fig. 1.
{Fig. 3} Fig. 3 is a diagram showing example data stored in an information storage portion provided in a manipulator control device of the medical manipulator system in Fig. 1.
{Fig. 4} Fig. 4 is a diagram showing bending angles of the flexible manipulator in Fig. 3 in a state in which the flexible manipulator is inserted to the descending colon.
{Fig. 5} Fig. 5 is a diagram showing bending angles of the flexible manipulator in Fig. 3 in a state in which the flexible manipulator is inserted to the transverse colon.

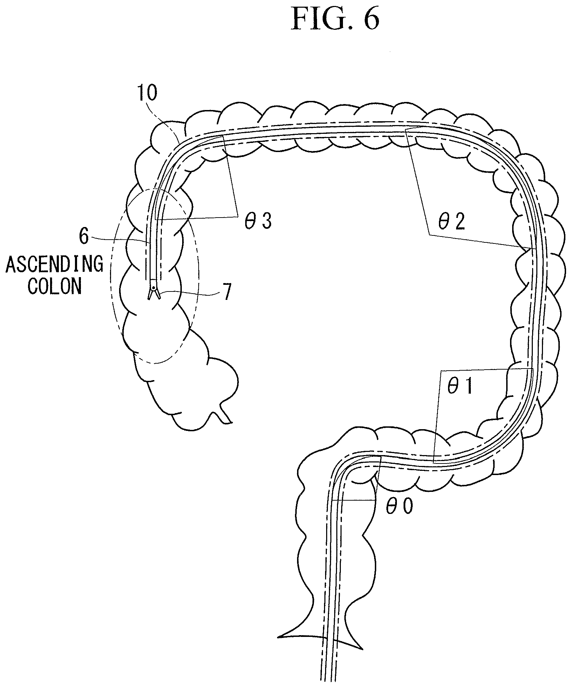

{Fig. 6} Fig. 6 is a diagram showing bending angles of the flexible manipulator in Fig. 3 in a state in which the flexible manipulator is inserted to the ascending colon.

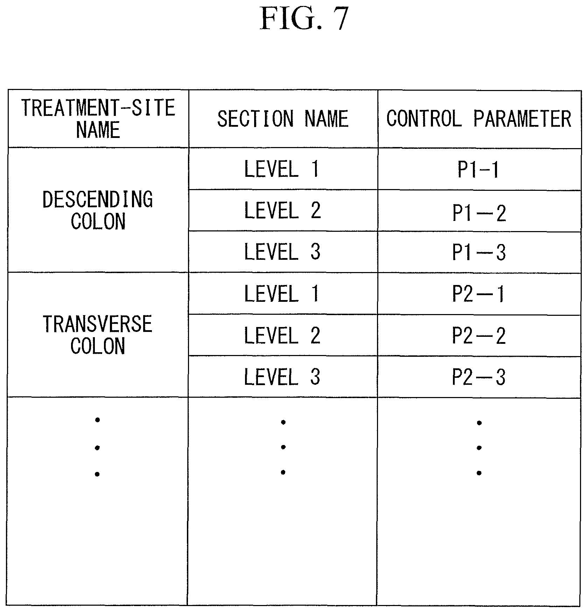

{Fig. 7} Fig. 7 is a diagram showing a modification of the example data stored in the information storage portion in Fig. 3.

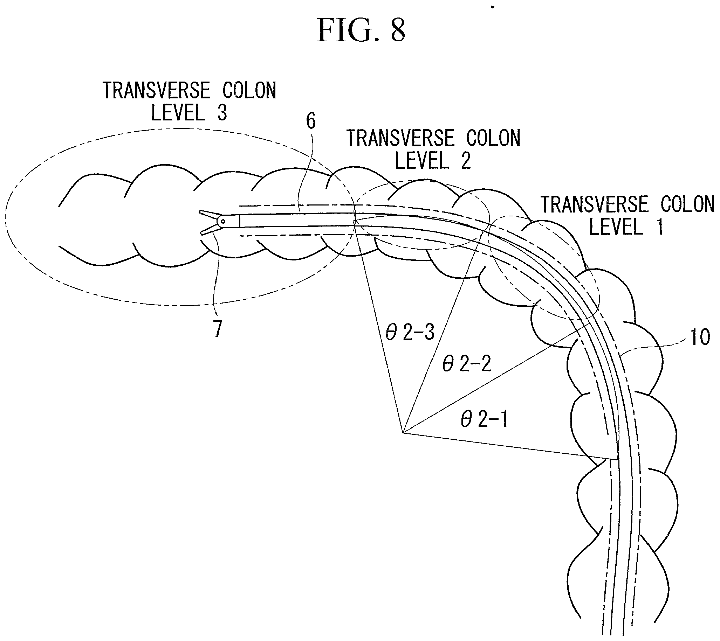

{Fig. 8} Fig. 8 is a diagram showing a modification for the case in which the transverse colon in Fig. 5 is specified as a treatment site.

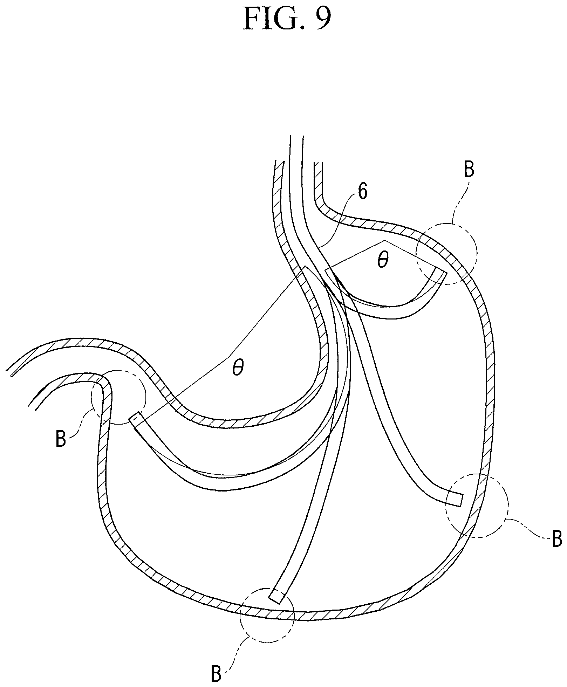

{Fig. 9} Fig. 9 is a diagram showing a modification for the case in which each portions of the stomach is specified as treatment site.

{Description of Embodiment}

A medical manipulator system 1 according to an embodiment of the present invention will be described below with reference to the drawings.

As shown in Fig. 1, the medical manipulator system 1 according to this embodiment is provided, for example, with: an operation input device (operation input portion) 2 operated by an operator A; a flexible manipulator 3 inserted into a body cavity of a patient P; a flexible-manipulator control device 4 that controls the flexible manipulator 3 according to the operation input to the operation input device 2; and a monitor 5.

The operation input device 2 includes a lever (not shown) operated by the fingers of the operator A. This operation input device 2 is a substantially similar input device having the same axial configuration as a gripping portion (movable portion) 7 arranged at a distal end of an insertion portion 6 of the flexible manipulator 3 to be described later, and compared to the gripping portion 7, for example, has a scale ratio of about 10-times. The operation input device 2 need not have a shape similar to the shape of the gripping portion 7, and the scale ratio is not limited to 10-times.

As shown in Fig. 2, the flexible manipulator 3 is provided with: the insertion portion 6 that is inserted into the body cavity of the patient P either directly or via a channel, an overtube (guide member) 10, or the like of a flexible endoscope inserted into the body cavity of the patient P; the gripping portion 7 that is arranged at a distal end of the insertion portion 6; a driving portion 8 that is arranged at a proximal end of the insertion portion 6; and a wire 9 that connects the driving portion 8 and the gripping portion 7 and moves the gripping portion 7 by a tensile force.

The gripping portion 7 is, for example, gripping forceps, and is configured to perform opening/closing operations by the tensile force of the wire 9. In this embodiment, in order to simplify the explanation, the gripping portion 7 will be described assuming that it has a single axis. The gripping portion 7 may have a plurality of joints.

The driving portion 8 is provided with a motor 15 and a conversion mechanism 11, such as a pulley, that converts a driving force of the motor 15 into the tensile force of the wire 9.

The wire 9 is arranged along a path formed in the insertion portion 6 and is configured so as to transmit the tensile force converted by the conversion mechanism 11 from the driving force generated by the motor 15 to the gripping portion 7.

As shown in Fig. 2, the flexible-manipulator control device 4 is provided with: an information input portion 12 inputting treatment-site specifying information for specifying a treatment target; an information storage portion (information control unit) 13 that stores the treatment-site specifying information input by the information input portion 12 and control parameters, in association with each other; and a control unit (drive control unit) 14 that controls the flexible manipulator 3 according to the operation input via the operation input device 2.

The information input portion 12 is an input device to which the operator A inputs the treatment-site specifying information for specifying the site to be treated. In this embodiment, treatment-site names are used as the treatment-site specifying information to be input. For example, the operation input device 2 displays candidates of sites to be treated on the monitor 5 in the form of characters or images, and is configured so that the treatment-site specifying information is input by allowing the operator A to select one of the candidates.

As shown in Fig. 3, the information storage portion 13 stores, in association with each other, a plurality of treatment-site names and control parameters P1 to P3 for the driving portion 8 that are appropriate for treating the respective treatment sites.

Examples of the control parameters P1 to P3 include master-slave scale ratios. A master-slave scale ratio is a parameter that determines how much an operation amount of the gripping portion 7 is to be set with respect to an operation amount of the operation input device 2.

In the case the insertion portion 6 of the flexible manipulator 3 is extended straight, by setting the master-slave scale ratio to be 0.1, it is possible to achieve an open/close amount of 1 mm in the gripping portion 7 when the lever of the operation input device 2 is moved by 10 mm. In addition, for example, by setting the master-slave scale ratio to be 0.2, because an open/close amount of 2 mm is achieved in the gripping portion 7 when the lever of the operation input device 2 is moved by 10 mm, the operator A should move the lever of the operation input device 2 by 5 mm in order to achieve the same open/close amount of 1 mm.

Here, in the case the insertion portion 6 of the flexible manipulator 3 is extended straight, the friction between the path formed in the insertion portion 6 of the flexible manipulator 3 and the wire 9 is the lowest, and thus, a force applied to the operation input device 2 is directly transmitted to the gripping portion 7 as it is as the tensile force acting on the wire 9. Therefore, in this case, when the master-slave scale ratio is 0.1, because the master-slave scale ratio is consistent with the actual scale ratio of the operation input device 2 and the gripping portion 7, it is possible to intuitively operate the gripping portion 7 via the operation input device 2, for example, while checking the gripping portion 7 with an endoscopic image.

However, in the case the insertion portion 6 of the flexible manipulator 3 is bent, friction occurs between the path formed in the insertion portion 6 and the wire 9 at each bent portion, and the friction is greater with an increase in the sum of angles (bending angles) at the bent portions. Because of this, the force applied to the operation input unit 2 transmitted the gripping portion 7 is difficult to be transmitted, and thus, if the master-slave scale ratio remains 0.1, the open/close amount of the gripping portion 7 becomes less than 1 mm due to elongation of the wire 9, even if the lever of the operation input device 2 is moved by 10 mm.

Therefore, by storing, as the control parameters, the master-slave scale ratios that increase as the sum of the bending angles of the insertion portion 6 increases, it becomes possible to achieve the same open/close amount in the gripping portion 7 by the same operation instruction input to the operation input device 2 regardless of the positions of the treatment sites.

Here, because the insertion path of the flexible manipulator 3 is substantially determined once the treatment site is specified, the bent shape of the insertion portion 6 of the flexible manipulator 3 when inserted along this insertion path, that is, the sum of the bending angles, is also substantially determined. Thus, the bent shape of the insertion portion 6 does not greatly change after the gripping portion 7 of the flexible manipulator 3 has reached the treatment site, and only the gripping portion 7 at the distal end is actuated.

Therefore, once the treatment site is determined, it is possible to unmistakably determine the optimal control parameter for treating this treatment site by the gripping portion 7. Also, because such control parameters are stored in the information storage portion 13 in association with the treatment site, it is possible to set the control parameter with which it is possible to accurately control the gripping portion 7 only by inputting the treatment-site name.

The control parameter is set, for example, by assuming, in advance, an insertion path for the case in which the insertion portion 6 is inserted into the body cavity so as to reach the individual sites in conformity to the body-cavity shape on the basis of the general anatomical structure of a human-body model, so that the value of the control parameter is set so as to increase as the sum of the bending angles $\theta$ of one or more bent portions included in the assumed insertion path increases.

For example, as shown in Figs. 4 to 6, the cases where treatment is performed to the descending colon, the transverse colon, or the ascending colon in the colon as an organ will be described as examples.

As shown in Fig. 4, in the case the treatment site is the descending colon, a sum $\theta_{all}$ of the bending angles $\theta_0$ and $\theta_1$ of two bent portions from the anus to the descending colon is $\theta_{all} = \theta_0 + \theta_1$.

As shown in Fig. 5, in the case the treatment site is the transverse colon, it is necessary to pass through one more bent portion, and thus, the sum $\theta_{all}$ of the bending angles is

$$\theta_{all} = \theta_0 + \theta_1 + \theta_2.$$

Furthermore, as shown in Fig. 6, in the case the treatment site is the ascending colon, it is necessary to further pass through one more bent portion from the transverse colon to the ascending colon, and thus, the sum $\theta_{all}$ of the bending angles is $\theta_{all} = \theta_0 + \theta_1 + \theta_2 + \theta_3$.

According to Euler's equation, tensile forces Tin and Tout that are applied to the wire 9 before and after a bent portion have the following relationship.

$$T_{out} = T_{in}e^{\mu\theta},$$

where $\mu$ is the friction coefficient of the wire 9, and $\theta$ is the bending angle.

In addition, because the wire 9 is stretched due to the difference between the tensile forces $T_{in}$ and $T_{out}$,

$$Kdx = T_{out} - T_{in},$$

where K is the spring constant of the wire 9, and dx is the elongation of the wire 9.

To summarize these expressions,

$$dx = (T_{in}(e^{\mu\theta}-1))/K,$$

and thus, it is understood that the elongation of the wire 9 is a function of the bending angle, and that it does not depend on the radius of curvature.

In other words, although the insertion portion 6 of the flexible manipulator 3 is bent at various radii of curvature in conformity to the shape of the body cavity when inserted into the body cavity, the radii of curvature in this case are not related to the elongation of the wire 9. Therefore, it is possible to determine the control parameter only on the basis of the sum of the bending angles.

The control unit 14 is configured so as to generate control signals for the driving portion 8 of the flexible manipulator 3 on the basis of the operation instruction input by the operator A via the operation input device 2 by using the control parameter read out from the information storage portion 13. For example, in the case the gripping portion 7 of the flexible manipulator 3 needs to be opened/closed by 1 mm via an operation instruction input by moving the lever of the operation input device 2 by 10 mm, the master-slave scale ratio, which serves as the control parameter, is set to be 0.1 in the state in which the insertion portion 6 is extended straight, and a greater master-slave scale ratio is set for treatment sites having a greater sum $\theta_{all}$ of the bending angles of the insertion portion 6. By doing so, it is possible to open/close the gripping portion 7 of the flexible manipulator 3 by 1 mm via the operation instruction input by moving the lever of the operation input device 2 by 10 mm, regardless of the bent state of the insertion portion 6.

The operation of the thus-configured medical manipulator system 1 according to this embodiment will now be described below.

In order to treat an affected potion that is located in the body cavity of the patient P by using the flexible manipulator 3 according to this embodiment, first, the operator A inputs, via the information input portion 12, the treatment-site name, for example, an organ name, at which the affected portion is located. For example, with the information input portion 12, a plurality of organ names are displayed on the monitor 5, and the operator A selects one of the organ names, thus inputting the organ name, which serves as the treatment-site name.

When the organ name is input, the control unit 14 searches the information storage portion 13 by using the input organ name as a key, and reads out and sets the control parameter stored therein in association with that organ name.

In this state, when the operator A inputs the operation instruction for the driving portion 8 by operating the operation input device 2, the control unit 14 generates control signals for controlling the driving portion 8 in accordance with the input operation instruction, and outputs the generated control signals to the driving portion 8.

In this case, the control unit 14 generates the control signal by using the control parameter read out from the information storage portion 13. In other words, in the above-described example, in the case the operation instruction input via the operation input device 2 is input by moving the lever by 10 mm and the read out master-slave scale ratio, which serves as the control parameter, is 0.1, the control signals to the driving portion 8 opens and closes the gripping portion 7 by 1 mm when the insertion portion 6 is extended straight.

On the other hand, in the case the master-slave scale ratio, which serves as the control parameter, is 0.2, the operation input device 2 needs to be operated by a greater amount because the insertion path of the insertion portion 6 is bent, and thus, the control parameter is set to be twice as great as the above-described value. Thus, by using this greater master-slave scale ratio, in this case also, the control signals for the driving portion 8 opens and closed the gripping portion 7 by 1 mm.

As has been described above, with the medical manipulator system 1 according to this embodiment, because an appropriate control parameter for the driving portion 8 for treating, by using the gripping portion 7, a treatment site specified by an organ name is set only by inputting that organ name, which serves as the treatment-site specifying information, it is not necessary to provide the insertion portion 6 with a sensor for detecting the bent state thereof, and thus, there is an advantage in that it is possible to achieve a size reduction and a cost reduction of the device by avoiding complicated signal processing.

In this embodiment, although an organ name, which is a treatment site, is selected as the treatment-site specifying information for specifying the treatment site, the treatment site is not limited to a specific organ, and it may be a symbol or the like defined in association with an organ. In addition, instead of selecting a displayed organ name, an organ may be selected from the displayed image.

In addition, in this embodiment, although an organ name is input as the treatment-site name, in addition thereto, with regard to an organ in which the size of the bending angles changes depending on the insertion depth of the insertion portion 6 even in the same organ, section names (for example, level 1, level 2, level 3, and so forth) sectioned on the basis of the insertion depth may be used together with the organ name, as shown in Fig. 7.

Specifically, as shown in Fig. 8, in the case a treatment site needs to be specified at an intermediate position of a bent portion extending from the descending colon to the transverse colon, a site at a portion that is bent from the descending colon by a bending angle $\theta$2-1 may be set to be level 1 of the transverse colon, a site at a portion that is further bent by a bending angle $\theta$2-2 may be set to be level 2 of the transverse colon, and a site at a portion that is even further bent by a bending angle $\theta$2-3 may be set to be level 2 of the transverse colon. By doing so, it is possible to specify the treatment site in detail, and it is possible to precisely set control parameters for the finely specified respective treatment sites. By doing so, it is possible to enhance the accuracy by which the gripping portion 7 is controlled.

In addition, the medical manipulator system 1 according to this embodiment may be provided with an endoscope having a channel through which the insertion portion 6 of the flexible manipulator 3 is made to pass or an overtube (guide member) 10. The endoscope or the overtube 10 generally has a greater diameter than that of the insertion portion 6 of the flexible manipulator 3 and has a higher rigidity. Therefore, when the endoscope or the overtube 10 is inserted into the body cavity, because bending occurs at a curvature in accordance with the rigidity of the endoscope or the overtube 10, insertion is made in conformity to the rough shape of the body cavity or partially in conformity to the shape of the body cavity without being affected by details of the shape of the body cavity.

In other words, when the soft insertion portion 6 of the flexible manipulator 3 is inserted into the body cavity by itself, the inserting portion 6 is made to twist so as to follow the detailed shape of the body cavity to reach the treatment site. But the endoscope or the overtube 10 having a higher rigidity than that of the insertion portion 6 is inserted so as to reach the treatment site at a curvature in accordance with its own rigidity, following the rough shape of the body cavity or, in some cases, while changing the shape of the body cavity. Thus, by guiding the insertion portion 6 so as to be inserted along the channel of the endoscope or the overtube 10 that has been inserted in such a manner, an advantage is afforded in that by suppressing changes in the insertion path, it is possible to accurately control the driving portion 8 by the control unit 14.

In addition, instead of the endoscope or the overtube 10, a member such as the wire 9 secured along the insertion portion 6 of the flexible manipulator 3 may be employed as the guide member.

In addition, in this embodiment, although the individual portions of the colon have been described as examples of the treatment sites, it is needless to say that, alternatively, the present invention may be applied to organs other than the colon, such as the oral/nasal cavity, blood vessels such as arteries, or the like.

Also, in addition to an organ, such as the colon or the like, for which it is possible to roughly determine the bent shape of the insertion portion 6 of the flexible manipulator 3, even with an organ that has a large space in the interior thereof, such as the stomach and the bladder, and in which the insertion portion 6 may take a plurality of bent shapes, it is possible to determine the bent form in the space to some extent on the basis of the relationship between the insertion direction and a treatment site B, as shown in Fig. 9. Therefore, in this case also, it is possible to apply the present invention in a similar manner.

In addition, in this embodiment, although the operator A is assumed to specify the treatment site by inputting the organ name or the like via the information input portion 12, alternatively, an actual insertion amount (insertion length) by which the insertion portion 6 of the flexible manipulator 3 is inserted into the body cavity may be input. The insertion amount can be read off a scale M that is provided, as shown in Fig. 2, on an outer surface of the insertion portion 6 of the flexible manipulator 3 or on an outer surface of the endoscope or the overtube 10 in the case the endoscope or the overtube 10 is employed, and the operator A may input the read numerical value. In this case, the information storage portion 13 may store the range of insertion amount and the control parameters in association with each other. In addition, in addition to keyboard input, dial input may be used for numerical value input.

In addition, in the case of inputting the insertion amount, for example, when a treatment site is located at a bent portion between the descending colon and the transverse colon, the sum of the bending angles of the insertion portion 6 increases with the insertion amount. In such a case, the bending angles thereof may be calculated on the basis of the input insertion amount, and the control parameter may be calculated on the basis of the calculated bending angles. Alternatively, an optimal control parameter may be calculated on the basis of the input insertion amount. By doing so, it is possible to change the control parameter to an optimal value in accordance with the insertion amount or the bending angles, and thus, it is possible to perform more precise control. In addition, the treatment-site specifying information is not limited to the insertion amount, and the control parameter may be changed on the basis of input information with which the bending angles can be specified, for example, (calculated) bending-angle information or the like read from an X-ray image of the patient P.

In addition, although it is assumed that the information storage portion 13 stores the treatment-site names and the control parameters in association with each other, alternatively, the treatment-site names and the sums of the bending angles may be stored in association with each other, and the control unit 14 may calculate a control parameter on the basis of a sum of the bending angles that is read out by using the treatment-site name as a key.

In particular, in the case the shape of the organ differs due to individual differences, the control unit 14 may calculate a sum of the bending angles obtained by correcting the bending angles due to the different portions, and may calculate the control parameter on the basis of the calculated sum of the bending angles. For example, in the case of a patient P that has an α-loop, a correction in which the bending angle corresponding to the α-loop is added may be performed.

{Reference Signs List}

    1 medical manipulator system
    2 operation input device (manipulation input portion)
    3 flexible manipulator
    6 insertion portion
    7 gripping portion (movable portion)
    8 driving portion
    9 wire (guide member)
    10 overtube (guide member)
    12 information input portion
    13 information storage portion (information control unit)
    14 control unit (drive control unit)
    A operator

**Claims**

1.   A medical manipulator system comprising:

        a flexible manipulator provided with an elongated flexible insertion portion, a movable portion that is provided at a distal end of the insertion portion, and a driving portion that is provided at a proximal end of the insertion portion and that drives the movable portion;
an operation input portion that is operated by an operator and with which an operation instruction for the flexible manipulator is input;
a drive control unit that controls the driving portion on the basis of the operation instruction input via the operation input portion;
an information input portion with which treatment-site specifying information for specifying a treatment target by using the flexible manipulator is input; and
an information control unit that sets a control parameter for controlling the driving portion on the basis of the treatment-site specifying information,
wherein the drive control unit controls the driving portion on the basis of the treatment-site specifying information input via the information input portion by using the control parameter set by the information control unit.

2. A medical manipulator system according to Claim 1, wherein the information control unit stores the treatment-site specifying information and the control parameter in association with each other, and the drive control unit controls the driving portion on the basis of the treatment-site specifying information input via the information input portion by using the control parameter read out from the information control unit.

3. A medical manipulator system according to Claim 1 or 2, further comprising:

   a guide member that has a greater rigidity than that of the insertion portion and that guides the insertion portion.

4. A medical manipulator system according to Claim 1 or 2, wherein the treatment-site specifying information includes a treatment-site name.

5. A medical manipulator system according to Claim 4, wherein the treatment-site name is an organ name.

6. A medical manipulator system according to Claim 4, wherein the treatment-site name is the organ name and a section name, the section that is sectioned in accordance with an insertion depth.

7. A medical manipulator system according to Claim 1 or 2, wherein the treatment-site specifying information is an inserted length of the insertion portion into a body of a patient.

8. A medical manipulator system according to Claim 1 or 2, wherein the treatment-site specifying information is a treatment-site name and an inserted length of the insertion portion into a body of a patient.

# FIG. 1

# FIG. 2

# FIG. 3

| TREATMENT-SITE NAME | CONTROL PARAMETER |
|---|---|
| DESCENDING COLON | P1 |
| TRANSVERSE COLON | P2 |
| ASCENDING COLON | P3 |
| ⋮ | ⋮ |

# FIG. 4

7

DESCENDING
COLON

6

θ1

10

θ0

# FIG. 5

TRANSVERSE
COLON
6
10
7
θ2
θ1
θ0

# FIG. 6

# FIG. 7

| TREATMENT-SITE NAME | SECTION NAME | CONTROL PARAMETER |
|---|---|---|
| DESCENDING COLON | LEVEL 1 | P1-1 |
| | LEVEL 2 | P1－2 |
| | LEVEL 3 | P1－3 |
| TRANSVERSE COLON | LEVEL 1 | P2－1 |
| | LEVEL 2 | P2－2 |
| | LEVEL 3 | P2－3 |
| ⋮ | ⋮ | ⋮ |

# FIG. 8

TRANSVERSE COLON
LEVEL 3

TRANSVERSE COLON
LEVEL 2

TRANSVERSE COLON
LEVEL 1

6

7

10

$\theta$ 2–3

$\theta$ 2–2

$\theta$ 2–1

EP 3 263 062 A1

# FIG. 9

## INTERNATIONAL SEARCH REPORT

| International application No. |
| --- |
| PCT/JP2016/053971 |

A. CLASSIFICATION OF SUBJECT MATTER
*A61B90/00*(2016.01)i

According to International Patent Classification (IPC) or to both national classification and IPC

B. FIELDS SEARCHED

Minimum documentation searched (classification system followed by classification symbols)
A61B34/00-34/37, A61B90/00

Documentation searched other than minimum documentation to the extent that such documents are included in the fields searched
Jitsuyo Shinan Koho         1922-1996    Jitsuyo Shinan Toroku Koho    1996-2016
Kokai Jitsuyo Shinan Koho   1971-2016    Toroku Jitsuyo Shinan Koho    1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2009-131374 A (Olympus Medical Systems Corp.), 18 June 2009 (18.06.2009), paragraphs [0014], [0045] to [0055]; fig. 1 & JP 4580973 B2 & CN 101444415 A & CN 101444415 B & EP 2064984 A2 & EP 2064984 A3 & EP 2064984 B1 & EP 2213221 A1 & US 2009/0143642 A1 paragraphs [0028], [0053] to [0068]; fig. 1 | 1-8 |
| A | WO 2014/136583 A1 (Olympus Corp.), 12 September 2014 (12.09.2014), paragraphs [0012] to [0026]; fig. 1 to 3 & CN 104885028 A & EP 2984535 A1 & JP 2016-505278 A paragraphs [0012] to [0026]; fig. 1 to 3 & US 2015/0245874 A1 | 1-8 |

[X] Further documents are listed in the continuation of Box C.          [ ] See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 04 April 2016 (04.04.16) | 19 April 2016 (19.04.16) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (January 2015)

**INTERNATIONAL SEARCH REPORT**

| International application No. |
| --- |
| PCT/JP2016/053971 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | WO 2015/012241 A1  (Olympus Corp.),<br>29 January 2015 (29.01.2015),<br>paragraphs [0022] to [0029]; fig. 1 to 3<br>& CN 105407827 A        & JP 2015-024036 A | 1-8 |
| A | JP 8-71072 A  (Olympus Optical Co., Ltd.),<br>19 March 1996 (19.03.1996),<br>paragraphs [0044], [0069] to [0071]<br>& US 6120433 A<br>column 10, lines 10 to 26; column 14, lines 16<br>to 57 | 1-8 |
| A | JP 2001-137257 A  (Olympus Optical Co., Ltd.),<br>22 May 2001 (22.05.2001),<br>paragraphs [0011] to [0021]; fig. 1 to 3<br>(Family: none) | 1-8 |
| A | US 2007/0083098 A1  (INTUITIVE SURGICAL INC.),<br>12 April 2007 (12.04.2007),<br>paragraph [0118]<br>& US 8079950 B2        & US 2012/0109377 A1<br>& US 8715167 B2        & US 2014/0296870 A1 | 1-8 |

Form PCT/ISA/210 (continuation of second sheet) (January 2015)

**EP 3 263 062 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4580973 B